# EUROPÄISCHE PATENTSCHRIFT

(11) **EP 3 906 070 B1**
(45) Veröffentlichungstag und Bekanntmachung des Hinweises auf die Patenterteilung: **25.09.2024**
(21) Anmeldenummer: 19836502.5
(22) Anmeldetag: 30.12.2019
(51) Int. Cl.: A61M 1/16, G01N 27/06

(54) **VORRICHTUNG UND VERFAHREN ZUR HERSTELLUNG EINER DIALYSELÖSUNG**
DEVICE AND METHOD FOR PRODUCING A DIALYSIS SOLUTION
DISPOSITIF ET PROCÉDÉ DE PRÉPARATION D'UNE SOLUTION DE DIALYSE

(30) Priorität: 31.12.2018 DE 102018010174
(43) Veröffentlichungstag der Anmeldung: 10.11.2021
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE)
(74) Vertreter: Herrmann, Uwe
(86) Internationale Anmeldenummer: PCT/EP2019/087163
(87) Internationale Veröffentlichungsnummer: WO 2020/141160

(56) Entgegenhaltungen:
- EP-A1- 2 962 711
- WO-A1-2016/169642
- DE-A1- 2 545 801
- US-A- 3 508 656

## Beschreibung

Die vorliegende Erfindung betrifft eine Vorrichtung zur Herstellung einer Dialyselösung, wobei die Vorrichtung eine Dialysatleitung zur Führung einer Dialyselösung sowie Mittel aufweist, die ausgebildet sind, eine Dialyselösung in der Dialysatleitung diskontinuierlich zu fördern, wobei die Vorrichtung einen Leitfähigkeitssensor aufweist, der angeordnet ist, die Leitfähigkeit der Dialyselösung zu messen, wobei die Vorrichtung eine stromaufwärts des Leitfähigkeitssensors an einer Zugabestelle in die Dialysatleitung mündende Konzentratleitung mit einer Konzentratpumpe aufweist sowie einen Konzentratbehälter, aus dem die Konzentratpumpe Konzentrat in die Konzentratleitung und aus dieser in die Dialysatleitung fördert.

Zur Durchführung einer Hemodialysebehandlung wird eine Dialyselösung in einem Filter bzw. Dialysator mittelbar, d.h. getrennt durch eine Membran mit dem zu behandelnden Blut eines Patienten in Kontakt gebracht. Dabei werden durch Diffusion und/oder durch Konvektion Stoffe zwischen dem Blut und der Dialyselösung ausgetauscht, wobei Schadstoffe aus dem Blut über die Membran in die Dialyselösung übergehen, wodurch das Überleben der Patienten mit Nierenversagen bzw. eingeschränkter Nierenfunktion gesichert wird.

Aus dem Stand der Technik ist es des Weiteren bekannt, die Dialyselösung dadurch herzustellen, dass RO-Wasser in dem hydraulischen Teil des Dialysegerätes, d.h. in dem nicht mit Blut beaufschlagten Teil des Dialysegerätes, mit einem sauren und mit einem basischen Konzentrat zu mischen und dabei die Konzentrate zu verdünnen, um die gebrauchsfertige Dialyselösung herzustellen.

Dabei wird die korrekte Zusammensetzung der Dialyselösung über Leitfähigkeitssensoren im Hinblick auf die Kompatibilität mit dem Patientenblut überwacht.

Üblicherweise erfolgen die Förderung der frischen Dialyselösung zu dem Dialysator und die Abfuhr von gebrauchter Dialyselösung von dem Dialysator mittels einer Bilanzierkammer, um ungewollte Volumenverschiebungen zwischen dem Patienten und dem hydraulischen Teil des Dialysegerätes zu vermeiden. Bekannte Bilanzierkammern bestehen aus zwei Doppelkammern, die jeweils durch eine Membran in zwei Hälften unterteilt sind. Während die eine Hälfte der Doppelkammer mit frischer Dialyselösung gefüllt wird, erfolgt eine Verdrängung von verbrauchtem Dialysat in der anderen Hälfte. Die Flusswege von und zu den Doppelkammern werden mittels Ventilen im Wechsel geöffnet und geschlossen. Durch die alternierende Befüllung der Bilanzierkammer ist der Dialysatfluss diskontinuierlich.

Dies hat den Nachteil, dass die Einspritzung der genannten Konzentrate in diesen diskontinuierlichen Fluss bei unzureichender Durchmischung der Komponenten zu Leitfähigkeitsschwankungen bzw. Konzentrationsschwankungen am Dialysator führen kann, was unerwünscht ist.

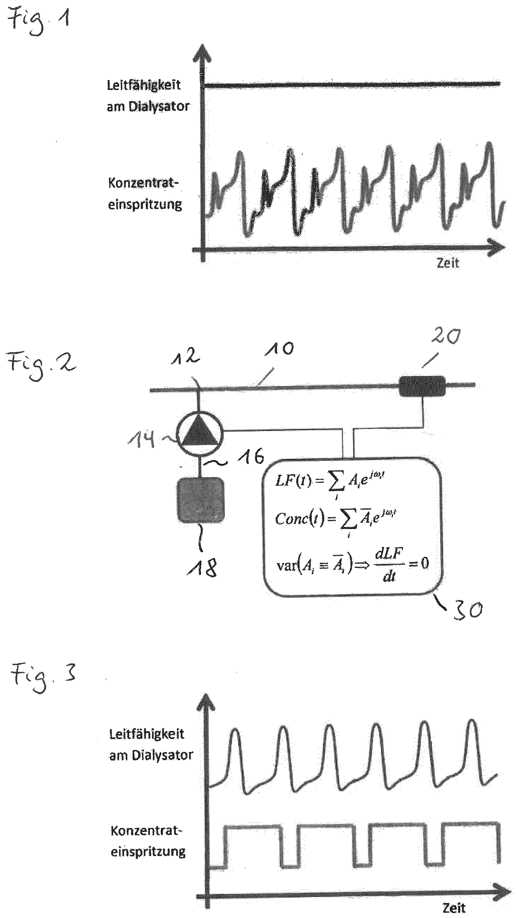

Ein solcher Zustand ist in Figur 3 gezeigt. Diese Figur zeigt den Verlauf der Leitfähigkeit am Dialysator über die Zeit sowie den Verlauf der Konzentrateinspritzung in die Dialysatleitung stromaufwärts des Dialysators.

Die aus Figur 3 ersichtlichen Leitfähigkeitsschwankungen sind die Folge einer fehlenden Synchronisation der Konzentratzufuhr mit dem diskontinuierlichen Volumenstrom.

Aus dem Stand der Technik ist es des Weiteren bekannt, in den genannten hydraulischen Kreislauf Mischkammern einzusetzen, um eine zeitlich konstante Leitfähigkeit am Dialysator zu erreichen.

Ein Nachteil bei dieser Vorgehensweise besteht jedoch darin, dass durch die Mischkammern das Füllvolumen des hydraulischen Kreislaufs deutlich erhöht wird, was zu verlängerten Reinigungszyklen bei der Desinfektion des hydraulischen Kreislaufs und zu längeren Wartezeiten bei der Stabilisierung der Leitfähigkeit nach einem Konzentrationswechsel während der Behandlung führt.

Die US 3 508 656 A, die WO 2016/169642 A1, die DE 25 45 801 A1 und die EP 2 962 711 A1 offenbaren Vorrichtungen und/oder Verfahren zur Herstellung einer Dialyselösung.

Der vorliegenden Erfindung liegt somit die Aufgabe zugrunde, eine Vorrichtung der eingangs genannten Art dahingehend weiterzubilden, dass mit vergleichsweise geringem apparativem Aufwand eine zeitlich stabile Leitfähigkeit der Dialyselösung am bzw. im Dialysator erhalten wird.

Diese Aufgabe wird durch eine Vorrichtung mit den Merkmalen des Anspruchs 1 gelöst. Danach ist vorgesehen, dass die Vorrichtung einen Controller bzw. eine Steuer- oder Regeleinheit aufweist, der mit dem Leitfähigkeitssensor sowie mit der Konzentratpumpe in Verbindung steht und die Konzentratpumpe derart ansteuert, dass die zeitlichen Leitfähigkeitsschwankungen in der Dialyselösung gegenüber einer kontinuierlichen Konzentratförderung verringert werden.

Der vorliegenden Erfindung liegt somit der Gedanke zugrunde, die Konzentratzufuhr so zu steuern, dass eine möglichst konstante Leitfähigkeit der Dialyselösung am bzw. im Dialysator erreicht wird. Die Leitfähigkeit wird gemessen und es erfolgt in Abhängigkeit des gemessenen Wertes eine Rückkopplung an die Konzentratpumpe dahingehend, dass diese in Abhängigkeit des gemessenen Leitfähigkeitswertes in ihrem Durchsatz und/oder hinsichtlich ihrer Betriebszeit und/oder -dauer eingestellt wird.

Die Leitfähigkeitsmessung dient somit dazu, die Konzentrateinspritzung so vorzunehmen bzw. so zu modulieren, dass eine stabile, d.h. zeitlich konstante oder im Wesentlichen zeitlich konstante Leitfähigkeit der dem Dialysator zugeführten Dialyselösung erhalten wird.

Findet eine Regelung statt, was von der Erfindung ebenfalls umfasst ist, ist die Regelgröße die Leitfähigkeit in der dem Dialysator zugeführten Dialyselösung und die Stellgröße der Zeitpunkt und/oder die Zeitdauer und/oder die Menge des zugeführten Konzentrats.

Ziel ist somit die Modulation der Konzentratzufuhr bzw. -einspritzung derart, dass eine zeitlich konstante oder weitgehend konstante Leitfähigkeit der Dialyselösung erhalten wird, die dem Dialysator zugeführt wird.

Dabei kann der Controller ausgebildet sein, die Konzentratpumpe so anzusteuern, dass die Leitfähigkeit dieser Dialyselösung über die Zeit konstant ist oder in einem Bereich von ± 1 - 5 % um den zeitlichen Mittelwert der Leitfähigkeit schwankt.

Erfindungsgemäß weist die Vorrichtung Mittel zur Spektralanalyse des Grundmusters der Leitfähigkeitsmodulation bei konstanter Konzentratzufuhr in die Dialyselösung auf, die ausgebildet sind, die elementaren Harmonischen der Leitfähigkeitsmodulation durch Spektralanalyse zu bestimmen.

Dabei ist der Controller ausgebildet, sukzessive für Frequenzen der Harmonischen die Konzentratpumpe so anzusteuern, dass die Leitfähigkeit in Phase und Amplitude so moduliert wird, dass der assoziierte harmonische Beitrag aus dem Leitfähigkeitsspektrum eliminiert ist.

Der Controller kann ausgebildet sein, mit diesem Vorgang mit der Frequenz der Harmonischen mit der größten Amplitude zu beginnen. Sukzessiv werden sodann die Frequenzen der nächsten Harmonischen mit den größten Amplituden gesucht.

Es erfolgt dann eine Modulation der Konzentratzufuhr, so dass die Beiträge im Spektrum der aus der Leitfähigkeit ermittelten Daten eliminiert sind.

Alternativ dazu kann vorgesehen sein, dass der Controller ausgebildet ist, dass dieser die Reihenfolge der Frequenzen beliebig wählt, also beispielsweise die beschriebene Vorgehensweise nicht zwingend mit der Frequenz der Harmonischen mit der größten Amplitude beginnt.

In einer alternativen Vorrichtung ist der Controller ausgebildet, die Konzentratpumpe nach einem Trial-and-Error-Prinzip ansteuern. Der Nachweis der korrekten Modulation der Konzentratzufuhr ist dann die weitgehende oder vollständige Konstanz der Leitfähigkeit am Dialysator über die Zeit.

Je mehr Beiträge zur Stabilisierung der Leitfähigkeit im diskontinuierlichen Fluss erforderlich sind, desto größer wird die Ordnung der sukzessiven Approximation.

Die vorliegende Erfindung betrifft des Weiteren ein Dialysegerät mit einer Vorrichtung nach einem der vorhergehenden Ansprüche.

Dabei werden die Mittel, die ausgebildet sind, eine Dialyselösung in der Dialysatleitung diskontinuierlich zu fördern, vorzugsweise durch die Bilanzkammer(n) des Dialysegerätes gebildet.

Die vorliegende Erfindung betriff des Weiteren ein Verfahren zur Herstellung einer Dialyselösung, die diskontinuierlich durch eine Dialysatleitung strömt, wobei der Dialyselösung ein Konzentrat zugeführt wird und wobei die Leitfähigkeit der Dialyselösung stromabwärts der Zugabestelle für das Konzentrat gemessen wird, wobei die Zufuhr von Konzentrat derart gesteuert wird, dass die zeitlichen Leitfähigkeitsschwankungen in der Dialyselösung gegenüber einer kontinuierlichen Konzentratförderung verringert werden.

Die Zufuhr von Konzentrat kann derart gesteuert werden, dass die Leitfähigkeit der Dialyselösung über die Zeit konstant ist oder in einem Bereich von ± 1 - 5 % um den zeitlichen Mittelwert der Leitfähigkeit schwankt.

Erfindugsgemäß ist vorgesehen, dass in einem ersten Schritt bei kontinuierlicher Konzentratzufuhr das Grundmuster der Leitfähigkeitsmodulation identifiziert wird und die elementaren Harmonischen der Leitfähigkeitsmodulation durch Spektralanalyse bestimmt werden und in einem zweiten Schritt sukzessive für Frequenzen der Harmonischen die Leitfähigkeit in Phase und Amplitude durch Änderung der Konzentratzufuhr so moduliert wird, dass der harmonische Beitrag aus dem Leitfähigkeitsspektrum eliminiert wird.

Der genannte zweite Schritt kann mit der Frequenz der Harmonischen mit der größten Amplitude begonnen werden. Alternativ ist es möglich, dass die Reihenfolge der Wahl der Harmonischen beliebig gewählt wird.

Auch ist es möglich, dass die Konzentratpumpe durch den Controller nach einem Trial-and-Error-Prinzip angesteuert wird, um die zeitlichen Leitfähigkeitsschwankungen der Dialyselösung zu eliminieren oder gegenüber einer Konzentratzufuhr, wie sie in Figur 3 dargestellt ist, zu verringern.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Auch wird darauf hingewiesen, dass der Begriff "Dialyselösung" weit zu verstehen ist und nicht nur die gebrauchsfertige Dialyselösung, wie sie vor dem Dialysator vorliegt, umfasst, sondern auch eine "unfertige" Lösung, in der nur eines von zwei oder mehreren Konzentraten vorliegen, bis hin zu dem Lösungsmittel, wie z.B. RO-Wasser, in das das oder die Konzentrate eingespritzt bzw. eingeführt werden.

Weitere Einzelheiten und Vorteile der Erfindung werden anhand eines in der Zeichnung dargestellten Ausführungsbeispiels näher erläutert.

Es zeigen:
- Figur 1:: eine regulierte, profilierte Konzentrateinspritzung und eine sich dabei ergebende stabile, d.h. zeitlich konstante Leitfähigkeit am Dialysator,
- Figur 2:: ein Schema zur Regelung bzw. Steuerung der Konzentrateinspritzung bzw. - zufuhr in einen diskontinuierlichen Permeatfluss und
- Figur 3:: eine ungeregelte Konzentrateinspritzung und eine instabile, d.h. zeitlich variable Leitfähigkeit am Dialysator.

Figur 1 zeigt den angestrebten Zustand einer zeitlichen stabilen, d.h. konstanten Leitfähigkeit der dem Dialysator zugeführten Dialyselösung.

Der in Figur 1 unten dargestellte Verlauf zeigt den Verlauf der Konzentrateinspritzung über die Zeit, wobei dieser Verlauf derart eingestellt wurde, dass sich trotz des diskontinuierlichen, d.h. zeitlich nicht konstanten Flusses der Dialyselösung eine konstante Leitfähigkeit in der Dialyselösung ergibt, die dem Dialysator zugeführt wird.

Zur Erreichung dieses Ziels, wird in die zum Dialysator führende Leitung 10 (vgl. Figur 2) an der Zugabestelle 12 in Flussrichtung der durch die Leitung strömenden Dialyselösung Konzentrat aus dem Reservoir 18 durch die Konzentratleitung 16 mittels der Pumpe 14 zugegeben. Stromabwärts dieser Zugabestelle 12 erfolgt die Messung der Leitfähigkeit mittels des Leitfähigkeitssensors 20.

Dessen Signale werden dem Controller zugeführt. In einem ersten Schritt wird bei kontinuierlicher Konzentrateinspritzung das Grundmuster der Leitfähigkeitsmodulation ermittelt und die elementaren Harmonischen dieser Modulation per Spektralanalyse bestimmt.

Beispielsweise beginnend mit der Frequenz der Harmonischen mit der größten Amplitude wird in Schritt 2 durch Modulation der Konzentratzufuhr die Leitfähigkeit moduliert in Amplitude und Phase, bis der assoziierte harmonische Beitrag aus dem Leitfähigkeitsspektrum verschwunden ist.

Sukzessiv werden die Frequenzen der nächsten Harmonischen mit den größten Amplituden gesucht, mit welchen die Konzentrateinspritzung moduliert wird, so dass die Beiträge im Spektrum der aus der Leitfähigkeit ermittelten Daten verschwunden sind.

Diese Vorgänge erfolgen im Controller 30, der in Abhängigkeit der Leitfähigkeitswerte eine entsprechende Signalisierung bzw. Ansteuerung der Konzentratpumpe 14 vornimmt.

Grundsätzlich ist darauf hinzuweisen, dass die Erfindung nicht auf die Zugabe genau eines Konzentrats beschränkt ist. Vielmehr ist von der Erfindung auch die Einspritzung mehrerer Konzentrate mit umfasst, auch wenn in Figur 3 nur die Zugabe eines Konzentrats gezeigt ist.

Deren Zugabe wird erfindungsgemäß so moduliert, dass sich eine zeitlich konstante oder im Wesentlichen zeitlich konstante Leitfähigkeit und damit eine konstante, d.h. zeitlich nicht schwankende Konzentratkonzentration in der dem Dialysator zugeführten Dialyselösung ergibt.

## Patentansprüche

1. Vorrichtung zur Herstellung einer Dialyselösung, wobei die Vorrichtung eine Dialysatleitung (10) zur Führung einer Dialyselösung sowie Mittel aufweist, die ausgebildet sind, eine Dialyselösung in der Dialysatleitung (10) diskontinuierlich zu fördern, wobei die Vorrichtung einen Leitfähigkeitssensor (20) aufweist, der angeordnet ist, die Leitfähigkeit der Dialyselösung zu messen, wobei die Vorrichtung eine stromaufwärts des Leitfähigkeitssensors (20) in die Dialysatleitung (10) an einer Zugabestelle (12) mündende Konzentratleitung (16) mit einer Konzentratpumpe aufweist sowie einen Konzentratbehälter (18), aus dem die Konzentratpumpe (14) Konzentrat in die Konzentratleitung (16) und aus dieser in die Dialysatleitung (10) fördert, wobei die Vorrichtung einen Controller (30) aufweist, der mit dem Leitfähigkeitssensor (20) sowie mit der Konzentratpumpe (14) in Verbindung steht und der ausgebildet ist, die Konzentratpumpe (14) derart anzusteuern, dass die zeitlichen Leitfähigkeitsschwankungen in der Dialyselösung stromabwärts der Zugabestelle (12) gegenüber einer kontinuierlichen Konzentratförderung verringert werden, **dadurch gekennzeichnet, dass** die Vorrichtung Mittel zur Spektralanalyse des Grundmusters der Leitfähigkeitsmodulation bei konstanter Konzentratzufuhr in die Dialyselösung aufweist, die ausgebildet sind, die Harmonischen der Leitfähigkeitsmodulation durch Spektralanalyse zu bestimmen und dass der Controller (30) ausgebildet ist, sukzessive für Frequenzen der Harmonischen die Konzentratpumpe (14) so anzusteuern, dass die Leitfähigkeit in Phase und Amplitude so moduliert wird, dass der harmonische Beitrag aus dem Leitfähigkeitsspektrum eliminiert ist.

2. Vorrichtung nach Anspruch 1, **dadurch gekennzeichnet, dass** der Controller (30) ausgebildet ist, die Konzentratpumpe (14) so anzusteuern, dass die Leitfähigkeit der Dialyselösung über die Zeit konstant ist oder in einem Bereich von ± 1 - 5 % um den zeitlichen Mittelwert der Leitfähigkeit schwankt.

3. Vorrichtung nach einem der vorhergehenden Ansprüche, **dadurch gekennzeichnet, dass** der Controller (30) ausgebildet ist, mit der Frequenz der Harmonischen mit der größten Amplitude zu beginnen.

4. Vorrichtung nach einem der vorhergehenden Ansprüche, **dadurch gekennzeichnet, dass** der Controller (30) ausgebildet ist, dass dieser die Reihenfolge der Frequenzen beliebig wählt.

5. Vorrichtung nach einem der vorhergehenden Ansprüche, **dadurch gekennzeichnet, dass** der Controller (30) ausgebildet ist, die Konzentratpumpe (14) nach einem Trial-and-Error-Prinzip ansteuern, um Leitfähigkeitsschwankungen der Dialyselösung stromabwärts der Zugabestelle (12) zu minimieren.

6. Dialysegerät mit einer Vorrichtung nach einem der vorhergehenden Ansprüche.

7. Dialysegerät nach Anspruch 6, **dadurch gekennzeichnet, dass** die Mittel, die ausgebildet sind, eine Dialyselösung in der Dialysatleitung (10) diskontinuierlich zu fördern, durch die Bilanzkammer des Dialysegerätes gebildet werden.

8. Verfahren zur Herstellung einer Dialyselösung, die diskontinuierlich durch eine Dialysatleitung (10) strömt, wobei der Dialyselösung ein Konzentrat zugeführt wird und wobei die Leitfähigkeit der Dialyselösung stromabwärts der Zugabestelle (12) für das Konzentrat gemessen wird, wobei die Zufuhr von Konzentrat derart gesteuert wird, dass die zeitlichen Leitfähigkeitsschwankungen in der Dialyselösung gegenüber einer kontinuierlichen Konzentratförderung verringert werden, **dadurch gekennzeichnet, dass** in einem ersten Schritt bei kontinuierlicher Konzentratzufuhr das Grundmuster der Leitfähigkeitsmodulation identifiziert wird und die Harmonischen der Leitfähigkeitsmodulation durch Spektralanalyse bestimmt werden und in einem zweiten Schritt sukzessive für Frequenzen der Harmonischen die Leitfähigkeit in Phase und Amplitude durch Änderung der Konzentratzufuhr so moduliert werden, dass der harmonische Beitrag aus dem Leitfähigkeitsspektrum eliminiert wird.

9. Verfahren nach Anspruch 8, **dadurch gekennzeichnet, dass** die Zufuhr von Konzentrat derart gesteuert wird, dass die Leitfähigkeit der Dialyselösung über die Zeit konstant ist oder in einem Bereich von ± 1 - 5 % um den zeitlichen Mittelwert der Leitfähigkeit schwankt.

10. Verfahren nach Anspruch 8 oder 9, **dadurch gekennzeichnet, dass** Schritt 2 mit der Frequenz der Harmonischen mit der größten Amplitude begonnen wird oder dass die Reihenfolge der Wahl der Harmonischen beliebig gewählt wird.

11. Verfahren nach einem der Ansprüche 8 bis 10, **dadurch gekennzeichnet, dass** die Konzentratpumpe (14) nach einem Trial-and-Error-Prinzip angesteuert wird, um die zeitlichen Leitfähigkeitsschwankungen der Dialyselösung zu eliminieren oder zu verringern.

## Claims

1. Apparatus for preparing a dialysis solution, wherein the apparatus has a dialyzate line (10) for conducting a dialysis solution and has means that are configured to discontinuously convey a dialysis solution in the dialyzate line (10), wherein the apparatus has a conductivity sensor (20) that is arranged to measure the conductivity of the dialysis solution, and wherein the apparatus has a concentrate line (16) opening into the dialyzate line (10) upstream of the conductivity sensor (20) at an addition point (12) and having a concentrate pump and has a concentrate container (18) from which the concentrate pump (14) conveys concentrate into the concentrate line (16) and from this into the dialyzate line (10), wherein the apparatus has a controller (30) that is connected to the conductivity sensor (20) and to the concentrate pump (14) and that is configured to control the concentrate pump (14) such that the conductivity fluctuations over time in the dialysis solution downstream of the addition point (12) are reduced with respect to a continuous concentrate conveying, **characterized in that** the apparatus has means for a spectral analysis of the basic pattern of the conductivity modulation with a constant concentrate supply into the dialysis solution, said means being configured to determine the harmonics of the conductivity modulation by spectral analysis; and **in that** the controller (30) is configured to successively control the concentrate pump (14) for frequencies of the harmonics such that the conductivity is modulated in phase and amplitude such that the harmonic contribution is eliminated from the conductivity spectrum.

2. Apparatus accordance with claim 1, **characterized in that** the supply of concentrate is controlled such that the conductivity of the dialysis solution over time is constant or fluctuates in a range from ± 1 - 5% about the mean time value of the conductivity.

3. Apparatus in accordance with one of the preceding claims, **characterized in that** the controller (30) is configured to start at the frequency of the harmonics with the greatest amplitude.

4. Apparatus in accordance with one of the preceding claims, **characterized in that** the controller (30) is configured such that it arbitrarily selects the order of the frequencies.

5. Apparatus in accordance with one of the preceding claims, **characterized in that** the controller (30) is configured to control the concentrate pump (14) in accordance with a trial and error principle to minimize conductivity fluctuations of the dialysis solution downstream of the addition point (12).

6. Dialysis machine having an apparatus in accordance with one of the preceding claims.

7. Dialysis machine in accordance with claim 6, **characterized in that** the means which are configured to discontinuously convey a dialysis solution in the dialyzate line (10) are formed by the balancing chamber of the dialysis machine.

8. Method of preparing a dialysis solution that flows discontinuously through a dialyzate line (10), wherein a concentrate is supplied to the dialysis solution, and wherein the conductivity of the dialysis solution is measured downstream of the addition point (12) for the concentrate, wherein the supply of concentrate is controlled such that the conductivity fluctuations over time in the dialysis solution are reduced with respect to a continuous concentrate conveying, **characterized in that** in a first step with a continuous concentrate supply, the basic pattern of the conductivity modulation is identified and the harmonics of the conductivity modulation are determined by spectral analysis and in a second step, the conductivity is successively modulated in phase and amplitude for frequencies of the harmonics by changing the concentrate supply such that the harmonic contribution is eliminated from the conductivity spectrum.

9. Method in accordance with claim 8, **characterized in that** the supply of concentrate is controlled such that the conductivity of the dialysis solution over time is constant or fluctuates in a range from ± 1 - 5% about the mean time value of the conductivity.

10. Method in accordance with claim 8 or 9, **characterized in that** step 2 is started at the frequency of the harmonics having the greatest amplitude; or **in that** the order of the selection of the harmonics is arbitrarily selected.

11. Method in accordance with one of claims 8 to 10, **characterized in that** the concentrate pump (14) is controlled in accordance with a trial and error principle to eliminate or reduce the conductivity fluctuations over time of the dialysis solution.

## Revendications

1. Dispositif de fabrication d'une solution de dialyse, dans lequel le dispositif présente une conduite de dialysat (10) destinée à guider une solution de dialyse ainsi que des moyens qui sont réalisés pour acheminer en discontinu une solution de dialyse dans la conduite de dialysat (10), dans lequel le dispositif présente un capteur de conductivité (20) qui est disposé pour mesurer la conductivité de la solution de dialyse, dans lequel le dispositif présente une conduite de concentré (16), débouchant sur un point d'ajout (12) dans la conduite de dialysat (10) en amont du capteur de conductivité (20), avec une pompe de concentré, ainsi qu'un contenant de concentré (18), duquel la pompe de concentré (14) achemine du concentré dans la conduite de concentré (16) et hors de celle-ci dans la conduite de dialysat (10), dans lequel le dispositif présente un organe de commande (30), qui est relié au capteur de conductivité (20) ainsi qu'à la pompe de concentré (14) et qui est réalisé pour piloter la pompe de concentré (14) de telle manière que les fluctuations de conductivité dans le temps dans la solution de dialyse sont réduite en aval du point d'ajout (12) par rapport à un acheminement continu de concentré, **caractérisé en ce que** le dispositif présente des moyens pour l'analyse spectrale du motif de base de la modulation de conductivité pour un apport de concentré constant dans la solution de dialyse, qui sont réalisés pour définir les harmoniques de la modulation de conductivité par analyse spectrale, et que l'organe de commande (30) est réalisé pour piloter la pompe de concentré (14) de manière successive pour des fréquences des harmoniques de telle sorte que la conductivité est modulée en phase et en amplitude de telle sorte que l'apport d'harmoniques est éliminé du spectre de conductivité.

2. Dispositif selon la revendication 1, **caractérisé en ce que** l'organe de commande (30) est réalisé pour piloter la pompe de concentré (14) de telle sorte que la conductivité de la solution de dialyse est constante dans le temps ou fluctue de la valeur moyenne dans le temps de la conductivité dans une plage de ± 1 - 5 %.

3. Dispositif selon l'une quelconque des revendications précédentes, **caractérisé en ce que** l'organe de commande (30) est réalisé pour commencer avec la fréquence des harmoniques avec la plus grande amplitude.

4. Dispositif selon l'une quelconque des revendications précédentes, **caractérisé en ce que** l'organe de commande (30) est réalisé pour sélectionner de manière arbitraire l'ordre des fréquences.

5. Dispositif selon l'une quelconque des revendications précédentes, **caractérisé en ce que** l'organe de commande (30) est réalisé pour piloter la pompe de concentré (14) selon un principe d'approximations successives pour minimiser des fluctuations de conductivité de la solution de dialyse en aval du point d'ajout (12).

6. Appareil de dialyse avec un dispositif selon l'une quelconque des revendications précédentes.

7. Appareil de dialyse selon la revendication 6, **caractérisé en ce que** les moyens, qui sont réalisés pour acheminer en discontinu une solution de dialyse dans la conduite de dialysat (10), sont formés par les chambres d'équilibrage de l'appareil de dialyse.

8. Procédé de fabrication d'une solution de dialyse, qui circule en discontinu à travers une conduite de dialysat (10), dans lequel un concentré est amené à la solution de dialyse et dans lequel la conductivité de la solution de dialyse est mesurée en aval du point d'ajout (12) pour le concentré, dans lequel l'apport de concentré est commandé de telle manière que les fluctuations de conductivité dans le temps dans la solution de dialyse sont réduites par rapport à un acheminement de concentré continu, **caractérisé en ce que** dans une première étape, lors d'un apport de concentré continu, le motif de base de la modulation de conductivité est identifié et les harmoniques de la modulation de conductivité sont définies par analyse spectrale, et dans une deuxième étape, la conductivité est modulée en phase et en amplitude en modifiant l'apport de concentré successivement pour des fréquences des harmoniques de telle sorte que l'apport d'harmoniques est éliminé du spectre de conductivité.

9. Procédé selon la revendication 8, **caractérisé en ce que** l'apport de concentré est commandé de telle manière que la conductivité de la solution de dialyse est constante dans le temps ou fluctue de la valeur moyenne dans le temps de la conductivité dans une plage de ± 1 - 5 %.

10. Procédé selon la revendication 8 ou 9, **caractérisé en ce que** l'étape 2 a commencé avec la fréquence des harmoniques avec la plus grande amplitude, ou que l'ordre de la sélection des harmoniques est choisi de manière arbitraire.

11. Procédé selon l'une quelconque des revendications 8 à 10, **caractérisé en ce que** la pompe de concentré (14) est pilotée selon un principe d'approximations successives pour éliminer ou réduire les fluctuations de conductivité dans le temps de la solution de dialyse.
